# FASCICULE DE BREVET EUROPEEN

(11) **EP 3 393 437 B1**
(45) Date de publication et mention de la délivrance du brevet: **07.06.2023**
(21) Numéro de dépôt: 16822424.4
(22) Date de dépôt: 15.12.2016
(51) Int. Cl.: A61K 8/37, A61Q 17/04, A61K 8/34, A61K 8/60

(54) **COMPOSITION PHOTOPROTECTRICE A BASE D'UN ESTER D'ACIDE CARBOXYLIQUE; UTILISATION DUDIT COMPOSE POUR AUGMENTER LE FACTEUR DE PROTECTION SOLAIRE**
LICHTSCHÜTZENDE ZUSAMMENSETZUNG AUF BASIS VON CARBOXYLSÄUREESTER, VERWENDUNG DER BESAGTEN VERBINDUNG ZUR ERHÖHUNG DES SONNENSCHUTZFAKTORS
PHOTOPROTECTIVE COMPOSITION BASED ON A CARBOXYLIC ACID ESTER; USE OF SAID COMPOUND FOR INCREASING THE SUN PROTECTION FACTOR

(30) Priorité: 22.12.2015 FR 1563156
(43) Date de publication de la demande: 31.10.2018
(73) Titulaire: L'OREAL, 75008 Paris (FR)
(72) Inventeur: WANG, Pamella, 94152 Chevilly la Rue (FR); TERRISSE, Isabelle, 94152 Chevilly la Rue (FR)
(74) Mandataire: L'Oreal
(86) Numéro de dépôt international: PCT/EP2016/081289
(87) Numéro de publication internationale: WO 2017/108588

(56) Documents cités:
- EP-A1- 0 881 896
- EP-A2- 0 427 411
- WO-A1-03/007906
- WO-A1-2015/181276
- WO-A2-01/21140
- DE-A1- 19 903 716
- DE-A1-102008 052 520
- US-A- 5 763 497
- US-B1- 6 207 140
- Seppic: "MOV 202/01/GB", , 23 mars 2001 (2001-03-23), XP055353407, Extrait de l'Internet: URL:http://www.biohope.com.cn/uploadfile/2 01011/20101119093739217.pdf [extrait le 2017-03-09]

## Description

La présente invention concerne une composition notament cosmétique et/ou dermatologique, destinée à la protection de la peau et/ou des cheveux contre le rayonnement ultraviolet, caractérisée par le fait qu'elle comprend, dans un milieu cosmétiquement acceptable au moins :
(a) un système photoprotecteur capable de filtrer le rayonnement UV ;
(b) un ou plusieurs esters d'acides carboxyliques comprenant au moins 12 atomes de carbone, ledit ester comprenant au moins 24 atomes de carbone ;
(c) au moins un alkylC₈-C₃₀(poly)glycoside, le ou les esters d'acides carboxyliques étant choisis parmi le béhénate de béhényle, l'arachidate d'arachidyle, et plus particulièrement le béhénate de béhényle.

La présente invention concerne également l'utilisation d'un ester d'acides carboxyliques comprenant au moins 12 atomes de carbone, ledit ester comprenant au moins 24 atome de carbone l'ester d'acide carboxylique étant choisi parmi le béhénate de béhényle, l'arachidate d'arachidyle, et plus particulièrement le béhénate de béhényle, pour augmenter le facteur de protection solaire (SPF) d'une composition comprenant dans un milieu cosmétiquement acceptable au moins un système photoprotecteur capable de filtrer le rayonnement UV.

Il est connu que les radiations de longueur d'onde comprise entre 280 nm et 400 nm permettent le bronzage de l'épiderme humain et que les radiations de longueur d'onde comprise entre 280 et 320 nm, connues sous le nom de rayons UV-B nuisent au développement du bronzage naturel. L'exposition est aussi susceptible d'induire une altération des propriétés biomécaniques de l'épiderme qui se traduit par l'apparition de rides conduisant un vieillissement prématuré de la peau. Ce rayonnement UV-B doit donc être filtré.

Il est aussi connu que les rayons UV-A de longueur d'onde comprise entre 320 et 400 nm pénètrent plus profondément dans la peau que les rayons UV-B. Les rayons UV-A provoquent un brunissement immédiat et persistant de la peau. Une exposition quotidienne aux rayons UVA, même de courte durée, dans des conditions normales peuvent conduire à une dégradation des fibres collagène et de l'élastine qui se traduit par une modification du microrelief de la peau, l'apparition de rides et une pigmentation irrégulière (tâches brunes, hétérogénéité du teint). Il est donc souhaitable de filtrer aussi le rayonnement UV-A.

De nombreuses compositions cosmétiques destinées à limiter l'assombrissement de la peau, améliorer la couleur et l'homogénéité du teint ont été proposées à ce jour. Il est bien connu dans le domaine des produits solaires, que de telles compositions peuvent être obtenues en utilisant des filtres UV, et en particulier des filtres UVB. Certaines compositions peuvent également contenir des filtres UVA. Ce système filtrant doit couvrir la protection UVB dans le but de limiter et contrôler la néo-synthèse de mélanine favorisant la pigmentation globale, mais doit aussi couvrir la protection UVA afin de limiter et contrôler l'oxydation de la mélanine déjà existante conduisant à l'assombrissement de la couleur de la peau.

De manière avantageuse, cette amélioration est particulièrement recherchée sur des peaux déjà pigmentées dans le but de ne pas augmenter ni la charge pigmentaire en mélanine ni la structure de la mélanine déjà présente au sein de la peau.

Aussi, il existe toujours un besoin important de trouver des produits solaires ayant un indice de protection suffisamment élevé. Les indices de protection élevés peuvent être atteints en incorporant plus de filtres à des concentrations élevées. Ceci n'est pas toujours réalisable malgré l'addition de quantités importantes de filtres. De plus de telles quantités peuvent entraîner des désagréments au niveau du confort (effet collant, rèche et/ou effet gras). En effet, les filtres solaires en particulier lipophiles apportant un effet gras non désiré.

EP 0 427 411 décrit des compositions topiques photoprotectrices comprenant des filtres UV et un ester d'acide gras.

EP 0 881 896 divulgue des méthodes de traitement cosmétique d'affections cutanées comprenant l'application sur la peau d'un ester d'acide salicylique et de rétinoide.

WO 03/007906 décrit des compositions comprenant des filtres UV organiques photostables possédant des propriétés antioxydantes.

DE 10 2008 052520, US 5,763,497 et WO 2015/181276 divulguent des compositions comprenant des esters, notamment d'acides gras.

Néanmoins, aucun de ces documents ne permet d'obtenir une composition stable avec un indice de protection élévé et de bonnes propriétés cosmétiques.

Il existe toujours un besoin de trouver des compositions suffisamment efficaces en photoprotection, qui présentent une bonne stabilité ainsi que de bonnes propriétés cosmétiques comme la facilité de l'étalement, un toucher non gras et bon glissant et qui ne présentent pas les inconvénients présentés ci-dessus.

Or, à la suite d'importantes recherches menées dans le domaine de la photoprotection évoqué ci-dessus, la Demanderesse a découvert de manière surprenante que l'addition d'un ester d'acide carboxylique particulier dans une composition contenant au moins un système filtrant les radiations UV, permettait d'augmenter son efficacité en photoprotection et en particulier son facteur de protection solaire.

De plus, les compositions selon l'invention permettent de diminuer l'effet gras du aux filtres UV, en particulier aux filtres lipophiles.

Cette découverte est à la base de la présente invention.

Ainsi, il est décrit de nouvelles compositions notamment cosmétiques en particulier destinées à la protection de la peau et/ou des cheveux contre le rayonnement ultraviolet, caractérisées par le fait qu'elles comprennent, dans un milieu notamment cosmétiquement acceptable au moins :
(a) un système photoprotecteur capable de filtrer le rayonnement UV ;
(b) un ou plusieurs esters d'acides carboxyliques comprenant au moins 12 atomes de carbone, ledit ester comprenant au moins 24 atomes de carbone.

Selon un premier aspect, l'invention concerne une composition notamment cosmétique, caractérisée par le fait qu'elle comprend, dans un milieu notamment cosmétiquement acceptable :
(a) au moins un système photoprotecteur capable de filtrer le rayonnement UV ;
(b) au moins un ester d'acide carboxylique comprenant au moins 12 atomes de carbone, ledit ester comprenant au moins 24 atomes de carbone ;
(c) au moins un alkylC8-C30(poly)glycoside, le ou les esters d'acides carboxyliques étant choisis parmi le béhénate de béhényle, l'arachidate d'arachidyle, et plus particulièrement le béhénate de béhényle.

Selon l'invention, on entend désigner de manière générale par « système photoprotecteur capable de filtrer le rayonnement UV », tout composé ou toute association de composés qui, par des mécanismes connus en soi d'absorption et/ou de réflexion et/ou diffusion du rayonnement UV-A et/ou UV-B, permet d'empêcher, ou du moins limiter, la mise en contact dudit rayonnement avec une surface (peau, cheveux,) sur laquelle ce ou ces composés ont été appliqués. En d'autres termes, ces composés peuvent être des filtres organiques photoprotecteurs absorbeurs d'UV ou des pigments minéraux diffuseurs et/ou réflecteurs d'UV, ainsi que leurs mélanges.

Par « cosmétiquement acceptable », on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Un autre objet encore de la présente invention réside dans l'utilisation d'au moins un ester d'acide carboxylique défini ci-dessous dans une composition comprenant dans un milieu cosmétiquement acceptable au moins un système photoprotecteur capable de filtrer le rayonnement UV, dans le but d'augmenter le facteur de protection solaire (SPF).

En d'autres termes, la présente invention concerne également l'utilisation d'au moins un ester d'acide carboxylique tel que défini ci-dessous pour augmenter le facteur de protection solaire (SPF) d'une composition comprenant dans un milieu cosmétiquement acceptable au moins un système photoprotecteur capable de filtrer le rayonnement UV.

Elle concerne également l'utilisation d'au moins un ester d'acide carboxylique tel que défini ci-dessous pour augmenter le facteur de protection solaire (SPF) d'une composition comprenant dans un milieu cosmétiquement acceptable au moins un système photoprotecteur capable de filtrer le rayonnement UV et au moins un alkylC₈-C₃₀(poly)glycoside.

Un autre objet de la présente invention est constitué par un procédé cosmétique de soin et/ou de maquillage d'une matière kératinique comprenant l'application sur la surface de ladite matière kératinique d'une composition selon l'invention telle que définie ci-dessus.

Elle concerne également un procédé cosmétique pour limiter l'assombrissement de la peau et/ou améliorer la couleur et/ou l'homogénéité du teint comprenant l'application sur la surface de la matière kératinique d'une composition telle que définie précédemment.

Elle concerne également un procédé cosmétique pour prévenir et/ou traiter les signes du vieillissement d'une matière kératinique comprenant l'application sur la surface de la matière kératinique d'une composition telle que définie précédemment.

### Définitions

Les définitions suivantes sont utilisées dans le présent texte.

Par « matières kératiniques humaines », on entend la peau (corps, visage, contour des yeux), cheveux, lèvres, muqueuses.

Par "physiologiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « milieu cosmétiquement acceptable », on entend un milieu compatible avec les matières kératiniques humaines, telle que la peau, les cheveux, les lèvres et les muqueuses.

Par « prévenir » ou « prévention », on entend selon l'invention le fait de réduire le risque de survenue ou de ralentir la survenue d'un phénomène donné, à savoir, selon la présente invention, les signes du vieillissement d'une matière kératinique.

On entend par « filtre UV » une molécule capable de filtrer le rayonnement UV entre 290 et 400nm.

Par « taille moyenne élémentaire », on entend la taille de particules non agrégées.

D'autres caractéristiques, aspects et avantages de la présente invention apparaîtront à la lecture de la description détaillée qui va suivre.

### ESTERS D'ACIDES CARBOXYLIQUES

Les esters d'acides carboxyliques comprenant au moins 24 atomes de carbone sont en particulier des esters comprenant deux ou trois chaînes grasses en C₁₂-C₃₀.

Le nombre total d'atomes de carbone de l'ester allant de 30 à 100, de préférence de 40 à 80.

Ils sont de préférence solides à une température inférieure ou égale à environ 30°C.

Les esters d'acides carboxyliques selon l'invention sont généralement présents dans les compositions selon l'invention dans des concentrations allant de 0,01 à 10 % en poids, de préférence de 0,1 à 10% en poids par rapport au poids total de la composition, plus préférentiellement de 0,5 à 5% en poids et encore plus préférentiellement de 1 à 3 % en poids.

### SYSTEME PHOTOPROTECTEUR

Selon l'invention, le système photoprotecteur peut être constitué par un ou plusieurs filtres organiques hydrophiles, lipophiles ou insolubles et/ou un ou plusieurs filtres inorganiques tels que des pigments minéraux. De manière préférentielle, il comprendra au moins un filtre UV organique hydrophile, lipophile ou insoluble.

Les filtres UV organiques sont notamment choisis parmi les dérivés cinnamiques ; les anthranilates ; les dérivés salicyliques ; les dérivés de dibenzoylméthane ; les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanine tels que ceux décrits dans les demandes WO04/006878, WO05/058269 et WO06/032741 et leurs mélanges.

Comme exemples d'agents photoprotecteurs organiques complémentaires, on peut citer ceux désignés ci-dessous sous leur nom INCI :

### Dérivés cinnamiques :

Ethylhexyl Methoxycinnamate vendu notamment sous le nom commercial PARSOL MCX par DSM NUTRITIONAL PRODUCTS,
Isopropyl Methoxycinnamate,
Isoamyl Methoxycinnamate vendu sous le nom commercial NEO HELIOPAN E 1000 par Symrise,
DEA Methoxycinnamate,
Diisopropyl Methylcinnamate,
Glyceryl Ethylhexanoate Dimethoxycinnamate.

### Dérivés de dibenzoylméthane :

Butyl Methoxydibenzoylmethane vendu notamment sous le nom commercial PARSOL 1789 par DSM,
Isopropyl Dibenzoylmethane.

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF.

### Dérivés salicyliques :

Homosalate vendu sous le nom « Eusolex HMS » par Rona/EM Industries,
Ethylhexyl Salicylate vendu sous le nom « NEO HELIOPAN OS » par Symrise,
Dipropyleneglycol Salicylate vendu sous le nom « DIPSAL » par SCHER,
TEA Salicylate, vendu sous le nom « NEO HELIOPAN TS » par Symrise,

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF.

### Dérivés de la benzophénone :

Benzophenone-1 vendu sous le nom commercial « UVINUL 400 » par BASF,
Benzophenone-2 vendu sous le nom commercial « UVINUL D50 » par BASF,
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5,
Benzophenone-6 vendu sous le nom commercial « Helisorb 11 » par Norquay,
Benzophenone-8 vendu sous le nom commercial « Spectra-Sorb UV-24 » par American Cyanamid,
Benzophenone-9 vendu sous le nom commercial« UVINUL DS-49» par BASF, Benzophenone-12,
2-(4-diethylamino-2-hydroxybenzoyl)-benzoate de n-hexyle vendu sous le nom commercial « UVINUL A+ » ou en mélange avec l'octylmethoxycinnamate sous le nom commercial « UVINUL A+B » par la société BASF.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX.

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par Symrise.

### Dérivés du phenyl benzotriazole :

Drometrizole Trisiloxane vendu sous le nom « Silatrizole » par RHODIA CHIMIE, Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS.

### Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine,
- la 2,4,6-tris-(4'-amino benzalmalonate de diisobutyle)-s- triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Dérivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par SYMRISE,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par DSM NUTRITIONAL PRODUCTS,

### Dérivés de 4,4-diarylbutadiène :

-1,1-dicarboxy (2,2'-diméthyl-propyl)-4,4-diphénylbutadiène,

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V, et leurs mélanges.

Les filtres organiques préférentiels sont choisis parmi :
Ethylhexyl Methoxycinnamate,
Ethylhexyl Salicylate,
Homosalate,
Butyl Methoxydibenzoylmethane,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle, 4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol,
Bis-Ethylhexyloxyphenol Methoxyphenyl Triazine,
Ethylhexyl triazone,
Diethylhexyl Butamido Triazone,
la 2,4,6-tris(4'-amino benzalmalonate de dinéopentyle)-s-triazine,
la 2,4,6-tris-(4'-amino benzalmalonate de diisobutyle)-s- triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine,
la 2,4,6-tris(terphenyl)-1,3,5-triazine,
Drometrizole Trisiloxane,
Polysilicone-15,
1,1-dicarboxy (2,2'-diméthyl-propyl)-4,4-diphénylbutadiène,
2,4-bis-[5-1(diméthylpropyl)benzoxazol-2-yl-(4-phenyl)-imino]-6-(2-ethylhexyl)-imino-1,3,5-triazine,
et leurs mélanges.

Les filtres UV inorganiques utilisés conformément à la présente invention sont des pigments d'oxyde métallique. Plus préférentiellement, les filtres UV inorganiques de l'invention sont des particules d'oxyde métallique ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm.

Ils peuvent être notamment choisis parmi les oxydes de titane, de zinc, de fer, de zirconium, de cérium ou leurs mélanges.

De tels pigments d'oxydes métalliques, enrobés ou non enrobés sont en particulier décrits dans la demande de brevet EP-A- 0 518 773. A titre de pigments commerciaux on peut mentionner les produits vendus les sociétés Kemira, Tayca, Merck et Degussa.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :
- de silice tel que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que les produits "MICROTITANIUM DIOXIDE MT 100 T, MT 100 TX, MT 100 Z, MT-01" de la société TAYCA, les produits "Solaveil CT-10 W" et "Solaveil CT 100" de la société UNIQEMA et le produit " Eusolex T-AVO" de la société MERCK,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR 351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS" de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA,
- de triéthanolamine tels que le produit "STT-65-S" de la société TITAN KOGYO,
- d'acide stéarique tel que le produit "TIPAQUE TTO-55 (C)" de la société ISHIHARA,
- d'hexamétaphosphate de sodium tel que le produit "MICROTITANIUM DIOXIDE MT 150 W" de la société TAYCA,
- le TiO₂ traité par l'octyl triméthyl silane vendu sous la dénomination commerciale "T 805" par la société DEGUSSA SILICES,
- le TiO₂ traité par un polydiméthylsiloxane vendu sous la dénomination commerciale "70250 Cardre UF TiO2SI3" par la société CARDRE,
- le TiO₂ anatase/rutile traité par un polydiméthylhydrogénosiloxane vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple :
- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple :
- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C₁₂-C₁₅) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C₁₂-C₁₅ avec polycondensat d'acide hydroxystéarique).

Les pigments d'oxyde de cérium non enrobés peuvent être par exemple ceux vendus sous la dénomination "COLLOIDAL CERIUM OXIDE" par la société RHONE POULENC.

Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ", "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Selon l'invention, les pigments d'oxyde de titane, enrobés ou non enrobés, sont particulièrement préférés.

Le système photoprotecteur selon l'invention est de préférence présent dans les compositions selon l'invention à une teneur allant de 0,1 % à 40 % en poids et en particulier de 5 à 25 % en poids, par rapport au poids total de la composition.

### ALKYLC₈-C₃₀(POLY)GLYCOSIDES

La composition selon l'invention comprend également au moins un alkylC₈-C₃₀(poly)glycoside.

Ces tensioactifs peuvent être plus particulièrement représentés par la formule générale suivante :

R₁O-(R₂O)ₜ (G)ᵥ

dans laquelle R₁ représente un radical alkyle et/ou alcényle linéaire ou ramifié comportant environ de 8 à 30 atomes de carbone, un radical alkylphényle dont le radical alkyle linéaire ou ramifié comporte de 8 à 24 atomes de carbone ; R₂ représente un radical alkylène comportant environ de 2 à 4 atomes de carbone ; G représente un motif sucre comportant de 5 à 6 atomes de carbone ; t désigne une valeur allant de 0 à 10, de préférence 0 à 4, de préférence 0 à 3 ; et v désigne une valeur allant de 1 à 15, de préférence de 1 à 4.

Il est en outre à noter que chaque unité de la partie polyoside de l'alkylpolyglycoside peut être sous forme isomérique α ou β, sous forme L ou D, et la configuration du reste saccharide peut être de type furanoside ou pyranoside.

Il est bien entendu possible d'utiliser des mélanges d'alkylpolyosides, susceptibles de différer les uns des autres par la nature du motif alkyle porté et/ou la nature de la chaîne polyoside porteuse.

Selon un mode de réalisation particulier, les alkyl(poly)glycosides sont des composés de formule décrite ci-dessus dans laquelle R₁ désigne plus particulièrement un radical alkyle saturé ou insaturé, linéaire ou ramifié comportant de 12 à 24 atomes de carbone, t désigne une valeur allant de 0 à 3 et plus particulièrement encore égale à 0, G peut désigner le glucose, le fructose, le galactose, maltose, maltotriose, lactose, cellobiose, mannose, ribose, dextrane, talose, allose, xylose, levoglucane, cellulose ou amidon, de préférence le glucose. Le degré de polymérisation, i.e. la valeur de v dans la formule ci-dessus, peut aller de 1 à 5, de préférence de 1 à 4. Le degré moyen de polymérisation est plus particulièrement compris entre 1 et 2,5, de préférence de 1,05 à 2,5, et plus préférentiellement de 1,1 à 2.

Les liaisons glucosidiques entre les motifs sucre sont de type 1-6 ou 1-4 et de préférence 1-4.

De préférence, on peut utiliser le coco(poly)glucoside (par ex le MONTANOV 82^{®} et le MONTANOV S^{®}), l'arachidyl(poly)glucoside (par ex le MONTANOV 202^{®}), le myristyl(poly)glucoside (par exemple le MONTANOV 14^{®}), le cétylstéaryl(poly)glucoside (par exemple le MONTANOV 68^{®}), les C₁₂-C₂₀ alkyl(poly)glucosides (par exemple le MONTANOV L^{®}), l'isostéaryl(poly)glucoside (par exemple le Montanov WO 18^{®}) ou l'octyldodécyl(poly)xyloside (par exemple le FLUIDANOV 20X ^{®}.

Selon un mode de réalisation particulier, la composition selon l'invention comprend en outre un alcool gras en C8-40.

Ainsi, selon un mode de réalisation préféré, l'alkylC₈-C₃₀(poly)glycoside est en mélange avec au moins un alcool gras.

On entend par alcool gras, un alcool aliphatique à longue chaîne comprenant de 8 à 40 atomes de carbone, et comprenant au moins un groupe hydroxyle OH.

De préférence, les alcools gras sont de structure R-OH avec R désignant un groupe alkyle linéaire, éventuellement substitué par un ou plusieurs groupes hydroxy, comprenant de 8 à 40, mieux de 10 à 30, voire de 12 à 24 atomes, encore mieux de 14 à 22 atomes de carbone.

Les alcools gras susceptibles d'être utilisés peuvent être choisis parmi, seul ou en mélange, l'alcool laurique ou laurylique (1 -dodécanol), l'alcool myristique ou myristylique (1 -tétradécanol), l'alcool cétylique (1 -hexadécanol), l'alcool stéarylique (1 -octadécanol), l'alcool arachidylique (1 -eicosanol), l'alcool béhénylique (1 -docosanol), l'alcool lignocérylique (1 -tetracosanol), l'alcool cérylique (1 -hexacosanol), l'alcool montanylique (1 -octacosanol), et l'alcool myricylique (1 -triacontanol).

Préférentiellement, l'alcool gras est choisi parmi l'alcool cétylique, l'alcool stéarylique, l'alcool arachidylique, l'alcool béhénylique et leurs mélanges, et encore plus préférentiellement parmi l'alcool arachidylique, l'alcool béhénylique et leurs mélanges.

Ainsi, on peut notamment utiliser le décylglucoside et le laurylglucoside commercialisés par exemple par la société Henkel sous les dénominations respectives PLANTAREN 2000 et PLANTAREN 1200, le cétostéarylglucoside éventuellement en mélange avec l'alcool cétostéarylique, commercialisé par exemple sous la dénomination MONTANOV 68 par la société SEPPIC, sous la dénomination TEGOCARE CG90 par la société Goldschmidt et sous la dénomination EMULGADE KE3302 par la société Henkel, ainsi que l'arachidyl glucoside, par exemple sous la forme du mélange d'alcools arachidique et béhénique et d'arachidylglucoside commercialisé sous la dénomination MONTANOV 202 par la société SEPPIC.

Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Plus préférentiellement, on utilisera l'arachidyl(poly)glucoside comme le produit commercial MONTANOV 202^{®} de SEPPIC.

En particulier, le ou les alkylC₈-C₃₀(poly)glycosides sont présents dans des concentrations allant de 0,01 % à 10 % en poids, de préférence de 0,1 % à 6 % en poids, et plus préférentiellement de 0,5 % à 4 % en poids, par rapport au poids total de la composition.

De préférence, le ou les alcools gras sont présents dans des concentrations allant de 0,01 % à 15 % en poids, de préférence de 0,1 % à 10 % en poids, et plus préférentiellement de 0,5 % à 5 % en poids, par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, des actifs cosmétiques ou dermatologiques, les émollients, les silicones, les agents anti-mousse, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras comme le benzoate d'alcools en C₁₂-C₁₅ vendu sous la dénomination commerciale « Finsolv TN » ou « Witconol TN » par la société WITCO, le Benzoate de 2-éthylphenyle comme le produit commercial vendu sous le nom X-TEND 226 ^{®} par la société ISP, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes, les trimellitates de trialkyle comme le trimellitate de tridécyle.

Comme composés cireux, on peut citer la cire de carnauba, la cire d'abeille, l'huile de ricin hydrogénée, les cires de polyéthylène et les cires de polyméthylène comme celle vendue sous la dénomination CIREBELLE 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le propanediol-1,3, le butylène glycol, le pentylène glycol, le dipropylène glycol ou le diéthylène glycol. Les alcools sont notamment les monoalcools en C1-C4 tels que par exemple l'éthanol, le propanol, l'isopropanol.

Les solvants organiques lorsqu'ils sont présents dans la composition de l'invention représentent de préférence de 0,01% à 50% en poids, préférentiellement de 1 % à 20% en poids par rapport au poids total de la composition.

Comme épaississants hydrophiles, on peut citer les polymères carboxyvinyliques tels que les Carbopols (Carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C₁₃-₁₄ isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose ; les polysaccharides et notamment les gommes telles que la gomme de Xanthane ; les dérivés siliconés hydrosolubles ou hydrodispersibles comme les silicones acryliques, les silicones polyéthers et les silicones cationiques et leurs mélanges.

Comme épaississants lipophiles, on peut citer les polymères synthétiques tels que les poly C₁₀-C₃₀ alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

La composition selon l'invention peut être aqueuse ou anhydre.

La teneur massique en eau de la composition est de préférence supérieure ou égale à 10%, avantageusement à 30%, préférentiellement à 40%, voire supérieure à 50% par rapport au poids total de la composition.

On entend par « composition anhydre » une composition contenant moins de 5% en poids d'eau par rapport au poids total de la composition, voire moins de 2 % en poids d'eau, et notamment exempte d'eau, l'eau n'étant pas ajoutée lors de la préparation de la composition mais correspondant à l'eau résiduelle apportée par les ingrédients mélangés.

Les compositions selon l'invention comprennent une phase grasse qui comprend au moins ledit ester d'acide carboxylique selon l'invention.

Au sens de l'invention, la phase grasse inclut tout corps gras liquides, généralement des huiles (aussi appelée phase grasse liquide ou phase huileuse), ou solides à l'image des cires.

Au sens de l'invention, une phase grasse liquide comprend au moins une huile. On entend par « *huile* », tout corps gras sous forme liquide à température ambiante à pression atmosphérique.

Une phase huileuse convenant à la préparation des compositions cosmétiques selon l'invention peut comprendre des huiles hydrocarbonées, siliconées, fluorées ou non, ou leurs mélanges.

Les huiles pourront être volatiles ou non volatiles.

Elles peuvent être d'origine animale, végétale, minérale ou synthétique. Selon une variante de réalisation, les huiles d'origine végétale sont préférées.

Au sens de la présente invention, on entend par « huile non volatile », une huile ayant une pression de vapeur inférieure à 0,13 Pa.

Au sens de la présente invention, on entend par « huile siliconée », une huile comprenant au moins un atome de silicium, et notamment au moins un groupe Si-O.

On entend par « *huile fluorée* », une huile comprenant au moins un atome de fluor.

On entend par « *huile hydrocarbonée* », une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyles ou acides.

Par « *huile volatile* », on entend, au sens de l'invention, toute huile susceptible de s'évaporer au contact de la peau en moins d'une heure, à température ambiante et pression atmosphérique. L'huile volatile est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1 300 Pa (0,01 à 10 mm de Hg).

### Huiles volatiles

Les huiles volatiles peuvent être hydrocarbonées, ou siliconées.

On peut notamment citer parmi les huiles volatiles hydrocarbonées ayant de 8 à 16 atomes de carbone les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'Isopars ou de Permetyls, les esters ramifiés en C₈-C₁₆ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isohexadécane.

On peut également citer les alcanes linéaires volatils comprenant de 8 à 16 atomes de carbone, en particulier de 10 à 15 atomes de carbone, et plus particulièrement de 11 à 13 atomes de carbone, par exemple tels que le n-dodécane (C₁₂) et le n-tétradécane (C₁₄) vendus par Sasol respectivement sous les références PARAFOL 12-97 et PARAFOL 14-97, ainsi que leurs mélanges, le mélange undécane-tridécane, les mélanges de n-undécane (C₁₁) et de n-tridécane (C₁₃) obtenus aux exemples 1 et 2 de la demande WO 2008/155059 de la Société Cognis, et leurs mélanges.

Comme huiles volatiles siliconées, on peut citer les huiles volatiles siliconées linéaires telles que l'hexamethyldisiloxane, l'octamethyltrisiloxane, le decamethyltetrasiloxane, le tetradecamethylhexasiloxane, l'hexadecamethylheptasiloxane et le dodecaméthylpentasiloxane.

Comme huiles volatiles siliconées cycliques, on peut citer l'hexamethylcyclotrisiloxane, l'octamethylcylotetrasiloxane, le decamethylcyclopentasiloxane et le dodecamethylcyclohexasiloxane.

### Huiles non volatiles

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale, les éthers de synthèse ayant de 10 à 40 atomes de carbone, comme le dicapryl ether,
- les esters de synthèse, comme les huiles de formule R₁COOR₂, dans laquelle R₁ représente un reste d'un acide gras linéaire ou ramifié comportant de 1 à 40 atomes de carbone et R₂ représente une chaîne hydrocarbonée, notamment, ramifiée contenant de 1 à 40 atomes de carbone à condition que R₁ + R₂ soit ≥ 10. Les esters peuvent être, notamment, choisis parmi les esters d'alcool et d'acide gras, comme par exemple, l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate d'isopropyle, le stéarate d'octyle, les esters hydroxylés, comme le lactate d'isostéaryle, l'hydroxystéarate d'octyle, les ricinoléates d'alcools ou de polyalcools, le laurate d'hexyle, les esters de l'acide néopentanoïque, comme le néopentanoate d'isodécyle, le néopentanoate d'isotridécyle, les esters de l'acide isononanoïque, comme l'isononanoate d'isononyle, l'isononanoate d'isotridécyle,
- les esters de polyols et les esters du pentaérythritol, comme le tétrahydroxystéarate/tétraisostéarate de dipentaérythritol,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique,
- les acides gras supérieurs en C₁₂-C₂₂, tels que l'acide oléique, l'acide linoléique, l'acide linolénique, et leurs mélanges,
- les huiles siliconés non phénylées, comme par exemple la caprylyl méthycone, et
- les huiles siliconés phénylées, comme par exemple les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, la triméthylpentaphényltrisiloxane, et leurs mélanges ; ainsi que les mélanges de ces différentes huiles.

De préférence, une composition selon l'invention comprend des huiles siliconées volatiles et/ou non volatiles.

Une composition selon l'invention peut comprendre de 5 % à 95 % en poids, mieux de 5 % à 40 % en poids, de préférence de 7 % à 35 % en poids d'huile(s) par rapport au poids total de ladite composition.

### Cires

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

A titre indicatif, une composition selon l'invention peut comprendre de 0,01 à 50 %, de préférence de 2 à 40 %, et mieux de 5 à 30 % en poids de cire(s) par rapport au poids total de la composition. Dans le cas des émulsions, la proportion de phase grasse sera choisie selon le sens de l'émulsion.

La phase grasse peut ainsi être présente dans la composition en une quantité allant de 1 % à 90 %, mieux allant de 5 % à 80 % et encore mieux de 10 % à 70 % en poids par rapport au poids total de la composition.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème ou qu'un lait ; sous la forme d'une lotion.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Les tensioactifs émulsionnants sont avantageusement distincts des alkylC₈-C₃₀(poly)glycosides considérés selon l'invention.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl diméthicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE O9 par la société Goldschmidt. On peut y ajouter aussi un ou plusieurs co-émulsionnants, qui, de manière avantageuse, peuvent être choisis dans le groupe comprenant les esters alkylés de polyol. On peut mentionner également l'OCTYLDODECANOL (AND) OCTYLDODECYLXYLOSIDE (FLUIDANOV 20X), POLYGLYCERYL-2 DIPOLYHYDROXYSTEARATE (DEHYMULS PGPH), POLYGLYCERYL-3 RICINOLEATE (AKOLINE PGPR), POLYGLYCERYL-3 DIISOSTEARATE (LAMEFORM TG1).

Comme esters alkylés de polyol, on peut citer notamment les esters de polyéthylèneglycol comme le PEG-30 Dipolyhydroxystearate tel que le produit commercialisé sous le nom ARLACEL P135 par la socité ICI ;

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination ARLACEL 987 par la société ICI ; l'isostéarate de sorbitan et le glycérol, tel que le produit commercialisé sous la dénomination ARLACEL 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stéarate commercialisé par exemple par la société ICI sous la dénomination ARLACEL 165 ; les éthers d'alcools gras oxyalkylénés (oxyéthylénés et/ou oxypropylénés) ; les esters de sucres comme le stéarate de sucrose ; les éthers d'alcool gras et de sucre. On peut citer également lécithines et dérivés (ex BIOPHILIC), les esters de sucres et le sodium stéaroyl lactylate.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1,4-cyclohexane-diméthanol (nom INCI : Polyester-5) vendu sous les dénominations "EASTMAN AQ POLYMER" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau du corps ou du visage et des lèvreset des cheveux, y compris le cuir chevelu, notamment pour la protection et/ou le soin de la peau et/ou des lèvres, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage, notamment de la peau, des lèvres, des ongles.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes.

L'homme du métier choisira le ou lesdits actifs en fonction de l'effet recherché sur la peau, les cheveux, les cils, les sourcils, les ongles et de préférence sur la peau.

La composition pourra comprendre en outre au moins un ingrédient tel que des charges à effet flouteur ou des agents favorisant la coloration naturelle de la peau, destiné à apporter un effet anti-âge immédiat visuel.

On peut citer notamment les agents matifiants, les charges à effet flouteur, les agents fluorescents, les agents favorisant la coloration naturellement rosée de la peau et les charges abrasives ou exfoliantes.

Pour complémenter et/ou optimiser les effets conférés par les actifs cosmétiques et/ou dermatologiques cités ci-dessus sur les matières kératiniques, il peut être avantageux d'intégrer dans les compositions de l'invention d'autres ingrédients additionnels.

En particulier, ces ingrédients additionnels pourront conférer un effet immédiat visuel qui sera relayé par l'effet biologique des actifs cités ci-dessus. Ils pourront également, via une action mécanique (ex : charges abrasvies), amplifier l'effet des actifs biologiques cités ci-dessus.

Ainsi la composition selon l'invention pourra comprendre en outre au moins un agent choisi parmi des agents matifiants supplémentaires, des charges à effet flouteur, des agents favorisant la coloration naturellement rosée de la peau, des charges abrasives ou agents exfoliants, et leurs mélanges.

### Agents matifiants

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 5 traduit généralement un effet matifiant.

L'agent matifiant pourra notamment être choisi parmi un amidon de riz ou un amidon de maïs nom INCI : ZEA MAYS (CORN) STARCH comme en particulier le produit vendu sous le nom commercial « FARMAL CS 3650 PLUS 036500 » par National Starch, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment :
- l'amidon de riz ou de maïs, en particulier un aluminium starch octenyl succinate commercialisé sous la dénomination Dry Flo^{®} par la société National Starch,
- la kaolinite ;
- les silices ;
- le talc ;
- un extrait de graines de potiron tel que commercialisé sous la dénomination Curbilene^{®} par la société Indena ;
- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon^{®}), de cellulose modifiée, de poly-p-phénylène téréphtamide, en acrylique, de polyoléfine, de verre, de silice, d'aramide, de carbone, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551^{®},
- des charges à effet optique telles que décrites dans la demande de brevet FR 2 869 796, en particulier :
- les poudres de polyamides (Nylon^{®}), comme par exemple les particules de Nylon 12 du type Orgasol d'Arkema de taille moyenne 10 microns et d'indice de réfraction 1,54,
- les poudres de silice, comme par exemple les Silica beads SB150 de Miyoshi de taille moyenne 5 microns et d'indice de réfraction 1,45,
- les poudres de polytétrafluoroéthylène, comme les PTFE ceridust 9205F de Clariant de taille moyenne 8 microns et d'indice de réfraction 1,36,
- les poudres de résine de silicone comme les Silicon resin Tospearl 145A de GE Silicone de taille moyenne 4,5 microns et d'indice de réfraction 1,41,
- les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100^{®} et F 80 ED^{®} de la société Matsumoto Yushi-Seiyaku,
- les poudres de cire comme les particules Paraffin wax microease 114S de micropowders de taille moyenne 7 microns et d'indice de réfraction 1,54,
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations
   "KSP-100", "KSP-101", "KSP-102", "KSP-103", "KSP-104", "KSP-105" par la société SHIN ETSU, et
- les poudres composites de talc/dioxyde de titane/alumine/silice comme celles vendues sous la dénomination Coverleaf^{®} AR-80 par la société Catalyst & chemicals,
- leurs mélanges,
- des composés absorbant et/ou absorbant le sébum tels que décrits dans la demande de brevet FR 2 869 796. On peut citer notamment :
- les poudres de silice, comme par exemple les microsphères de silice poreuses vendues sous la dénomination "SILICA BEADS SB-700" commercialisées par la société MYOSHI, les "SUNSPHERE^{®} H51", "SUNSPHERE^{®} H33" , "SUNSPHERE^{®} H53" commercialisées par la société ASAHI GLASS ; les microsphères de silice amorphe enrobées de polydiméthylsiloxane vendues sous la dénomination "SA SUNSPHERE^{®} H-33" et "SA SUNSPHERE^{®} H-53" commercialisées par la société ASAHI GLASS ;
- les poudres de silicates mixtes amorphes, notamment d'aluminium et de magnésium, comme par exemple celle commercialisée sous la dénomination "NEUSILIN UFL2" par la société Sumitomo.
- les poudres de polyamides (nylon^{®}), comme par exemple "l'ORGASOL^{®} 4000" commercialisé par la société Arkema, et
- les poudres de polymères acryliques, notamment de polyméthacrylate de méthyle, comme par exemple le "COVABEAD^{®} LH85" commercialisé par la société WACKHERR ; de polyméthacrylate de méthyle/diméthacrylate d'éthylène glycol, comme par exemple le "DOW CORNING 5640 MICROSPONGE^{®} SKIN OIL ADSORBER" commercialisé par la société DOW CORNING, ou le "GANZPEARL^{®} GMP-0820" commercialisé par la société GANZ CHEMICAL ; de polyméthacrylate d'allyle/diméthacrylate d'éthylène glycol, comme par exemple le "POLY-PORE^{®} L200" ou le "POLY-PORE^{®} E200" commercialisés par la société AMCOL ; de copolymère diméthacrylate d'éthylène glycol/méthacrylate de lauryle, comme par exemple le "POLYTRAP^{®} 6603" commercialisé de la société DOW CORNING ;
   - les particules de silicate, telle que la silicate d'alumine ;
   - les particules de silicates mixtes, telles que :
   - les particules de silicate d'aluminium et de magnésium, telles que la saponite ou silicate de magnésium et d'aluminium hydraté avec un sulfate de sodium commercialisée sous la dénomination commerciale Sumecton^{®} par la société Kunimine ;
   - le complexe silicate de magnésium, hydroxyéthylcellulose, huile de cumin noir, huile de courge et phospholipides ou Matipure^{®} de Lucas Meyer, et
   - leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyethylènes, les poudres de copolymères acryliques, les microsphères de copolymères acryliques expansées, les microbilles de résines de silicones, les particules de silicate mixte et leurs mélanges.

### Charges à effet flouteur

Ces charges peuvent être tout matériau susceptible de modifier les rides par ses propriétés physiques intrinsèques et de les masquer. Ces charges peuvent notamment modifier les rides par un effet tenseur, un effet de camouflage, ou un effet de floutage.

En tant que charge, on peut donner à titre d'exemples les composés suivants :
- les microparticules poreuses de silice comme par exemple les Silica Beads^{®} SB 150 et SB 700 de Myochi de taille moyenne de 5µm et les SUNSPHERES^{®} série H d'Asahi Glass comme les H33, H51 de taille respectivement de 3,5 et 5 µm.
- les particules hémisphériques creuses de résines de silicones comme les NLK 500^{®}, NLK 506^{®} et NLK 510^{®} de Takemoto Oil and Fat, notamment décrites dans EP-A-1579849,
- les poudres de résine de silicone comme par exemple les SILICON Resin Tospearl^{®} 145 A DE GE silicone de taille moyenne de 4,5µm.
- les poudres de copolymères acryliques, notamment de poly(meth)acrylate de méthyle comme par exemple les particules PMMA Jurimer MBI^{®} de Nihon Junyoki de taille moyenne de 8µm, les sphères creuses de PMMA vendues sous la dénomination COVABEAD^{®} LH 85 par la société Wackherr et les microsphères de vinylidène/acrylonitrile/méthacrylates de méthylène expansées vendues sous la dénomination Expancel^{®}.
- les poudres de cires comme les particules Paraffin wax microloase^{®} 114S de Micropowders de taille moyenne de 7µm.
- les poudres de polyéthylènes notamment comprenant au moins un copolymère éthylène/acide acrylique comme par exemple les FLOBEADS^{®} EA 209 E de Sumimoto de taille moyenne de 10µm.
- les poudres d'organopolysiloxanes élastomériques réticulées enrobées de résine de silicone notamment de silsesquioxane sous la dénomination KSP 100^{®}, KSP 101^{®}, KSP 102^{®}, KSP 103^{®}, KSP 104^{®} et KSP 105^{®} par la société Shin Etsu.
- les poudres composites de talc/dioxyde ou de titane/alumine/silice comme par exemple les Coverleaf AR 80^{®} de la société Catalyst & Chemical.
- le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.
- les fibres hydrophiles ou hydrophobes synthétiques ou naturelles, minérales ou organiques telles que des fibres de soie, de coton, de laine, de lin, de cellulose extraites notamment du bois, des légumes ou des algues, de polyamide (Nylon^{®}), de cellulose modifiée, de poly-p-phénylène téréphtamide, en acrylique, de polyoléfine, de verre, de silice, d'aramide, de carbone, de polytétrafluoroéthylène (Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742.
- les silicones réticulés élastomères sphériques comme comme les Trefil E-505C^{®} ou E-506 C^{®} de chez Dow Corning.
- les charges abrasives qui par effet mécanique apportent un lissage du microrelief cutané, telles que la silice abrasive comme par exemple Abrasif SP^{®} de Semanez ou des poudres de noix ou de coques (abricot, noix par exemple de Cosmétochem).

Les charges ayant un effet sur les signes du vieillissement sont notamment choisies parmi des microparticules poreuse de silice, des particules hémisphériques creuses de silicones, des poudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.

La charge peut être une charge « soft focus ».

Par charge « soft-focus », on entend une charge qui en plus donne de la transparence au teint et un effet flou. De préférence, les charges « soft-focus » ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/TiO2 ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3^{®} par la société Nippon Talc, la poudre de Nylon^{®} 12, notamment celle vendue sous le dénomination Orgasol 2002 Extra D Nat Cos^{®} par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53^{®} par la société Asahi Glass, et les micro-billes de silice telles que celles vendues sous la dénomination SB-700^{®} ou SB-150^{®} par la société Miyoshi, cette liste n'étant pas limitative.

La concentration de ces charges ayant un effet sur les signes du vieillissement dans les compositions selon l'invention peut être comprise entre 0,1 et 40 %, voire entre 0,1 et 20 % en poids par rapport au poids total de la composition.

Les exemples qui suivent servent à illustrer l'invention sans toutefois présenter un caractère limitatif. Dans ces exemples, les quantités des ingrédients compositions sont données en % pondéral par rapport au poids total de la composition.

### EXEMPLES 1 à 3 :

On a préparé des formulations contenant les ingrédients suivants.

| | 1 (invention) | 2 | 3 |
|---|---|---|---|
| SODIUM POLYACRYLOYLDIMETHYL TAURATE (AND) POLYSORBATE 80 (AND) SORBITAN OLEATE (SIMULGEL 800 - SEPPIC) | 2 | 2 | 2 |
| OCTOCRYLENE (UVINUL N539 - BASF) | 7 | 7 | 7 |
| ARACHIDYL ALCOHOL (AND) BEHENYL ALCOHOL (AND) ARACHIDYL GLUCOSIDE (MONTANOV 202 - SEPPIC) | 4 | 4 | 4 |
| HOMOSALATE | 5 | 5 | 5 |
| BUTYL METHOXYDIBENZOYL METHANE | 3 | 3 | 3 |
| ETHYLHEXYL SALICYLATE | 5 | 5 | 5 |
| PHENOXYETHANOL | 0,5 | 0,5 | 0,5 |
| **BEHENYL BEHENATE** | 1 | 0 | 0 |
| CIRE DE POLYMETHYLENE (CIREBELLE 303) | 0 | 1 | 0 |
| EAU QSP | 100,0 | 100,0 | 100,0 |

Pour chacune des compositions 1 à 3, on a ensuite déterminé le facteur de protection solaire (SPF) qui lui était attaché. Celui-ci a été déterminé en utilisant la méthode in vitro décrite par V. Wandel et al. Dans SOFW Journal 127 (2001) ; cette méthode consiste à déterminer les facteurs de protection monochromatiques dans une gamme de longueurs d'onde de 290 à 400 nm et à calculer à partir de ceux-ci le facteur de protection solaire selon une équation mathématique donnée. La mesure a été réalisée avec un pas de 1 nm sur un appareil UV-1000S de la société Labsphere, 0,6 mg/cm² de produit étant étalé sur une plaque de PMMA dépolie. Les résultats (valeur moyenne correspondant 5 plaques par produit, 10 points par plaque) sont regroupés dans le tableau (I) ci-dessous :

### Mesure de la matité par évaluation in vitro

On a mesuré la matité obtenue avec la composition 1 selon l'invention, et avec les compositions 2 et 3 données à titre d'exemple comparatif, en utilisant une carte de contraste (Prufkarte type 24/5 - 250 cm²) commercialisée par la société ERICHSEN. La composition a été étalée à raison de 2 mg/cm² à l'aide d'un tire-film mécanique pour obtenir un film d'une épaisseur de 100 microns. Les cartes sont stockées 24 h à 37°C sous atmosphère contrôlée entre 15% et 25% d'humidité.

On a effectué 1 vaporisation d'un mélange (20% acide oléique + 80% eau de Vichy + 1% OLETH-10), la quantité déposée est d'environ 0,3 g par pulvérisation. On a fait une mesure à T0 de la réflexion à l'aide d'un gonioréflectomètre (UB T0 puis on a attendu 6 minutes à température ambiante puis on fait une nouvelle mesure (UB T6mn). Le résultat obtenu est le rapport R entre la réflexion spéculaire et la réflexion diffuse (unité de brillance). La valeur de R est d'autant plus faible que l'effet matifiant est important.

Le tableau (I) ci-dessous donne les valeurs d'unité de brillance immédiate (UBT0) et après pulvérisation d'une solution artificielle de sébum/sueur (UBT6min)

**Tableau (I)**

| | Matité immédiateT0 | Matité après vaporisation de solution de sébum artificiel /sueur T6mn | SPF in vitro |
|---|---|---|---|
| Base SPF + 1% behenyl behenate (Ex 1) | 2,8 | 3,55 | 22,5 |
| Base SPF + 1% cirebelle 303 (Ex 2) | 3,25 | 11,45 | 15,38 |
| Base SPF (Ex 3) | 3,23 | 9,6 | 14,3 |

### Exemples 4 à 6

On a préparé les 3 compositions suivantes :

| | | | |
|---|---|---|---|
| | 4 | 5 | 6 |
| ARACHIDYL ALCOHOL (AND) | 3 | 3 | 3 |

| BEHENYL ALCOHOL (AND) ARACHIDYL GLUCOSIDE (MONTANOV 202 - SEPPIC) | | | |
|---|---|---|---|
| OCTOCRYLENE | 7 | 7 | 7 |
| PARFUM | 0,1 | 0,1 | 0,1 |
| DISODIUM EDTA | 0,1 | 0,1 | 0,1 |
| BUTYL METHOXYDIBENZOYL METHANE | 3 | 3 | 3 |
| SILICA (and) TITANIUM DIOXIDE | 3 | 3 | 3 |
| PARAFFIN | 0,5 | 0,5 | 0,5 |
| ISONONYL ISONONANOATE | 2,5 | 2,5 | 2,5 |
| PHENOXYTHANOL | 0,5 | 0,5 | 0,5 |
| DIMETHICONE | 3 | 3 | 3 |
| GLYCERIN | 5 | 5 | 5 |
| BEHENYL BEHENATE | 0,5 | 1 | 0 |
| AMMONIUM ACRYLOYLDIMETHYLTAURATE /VP COPOLYMER (ARISTOFLEX AVC - CLARIANT | 0,5 | 0,5 | 0,5 |
| DICAPRYLYL CARBONATE | 5 | 5 | 5 |
| EAU QSP | 66,3 | 66,3 | 66,3 |

Le SPF des compositions 4, 5 et 6 a été mesuré *in vivo* selon la méthode ISO 24444 (2010).

Le SPF des compositions selon l'invention comprenant le béhénate de béhényle est nettement supérieur à celui de la composition n'en contenant pas.

| | **SPF IN VIVO** |
|---|---|
| Base SPF 2 (Ex 6) comparatif | 12 |
| Base SPF 2 + 0,5% behenyl behenate (Ex 4) Invention | **16** |
| Base SPF 2 + 1% behenyl behenate (Ex 5) Invention | **19** |

## Revendications

1. Composition notamment cosmétique, **caractérisée par le fait qu'**elle comprend, dans un milieu notamment cosmétiquement acceptable :
(a) au moins un système photoprotecteur capable de filtrer le rayonnement UV ;
(b) au moins un ester d'acide carboxylique comprenant au moins 12 atomes de carbone, ledit ester comprenant au moins 24 atomes de carbone ;
(c) au moins un alkylC₈-C₃₀(poly)glycoside,
le ou les esters d'acides carboxyliques étant choisis parmi le béhénate de béhényle, l'arachidate d'arachidyle, et plus particulièrement le béhénate de béhényle.

2. Composition selon la revendication 1, où le ou les esters d'acides carboxyliques sont présents dans des concentrations allant de 0,01 à 10 % en poids et encore plus préférentiellement de 0,5 à 5 % et encore plus particulièrement de 1 à 3 % par rapport au poids total de la composition.

3. Composition selon l'une quelconque des revendications 1 à 2, où le ou les alkylC₈-C₃₀(poly)glycosides sont choisis parmi le coco(poly)glucoside, l'arachidyl(poly)glucoside, le myristyl(poly)glucoside, le cétylstéaryl(poly)glucoside, les C₁₂-C₂₀ alkyl(poly)glucosides, l'isostéaryl(poly)glucoside et l'octyldodécyl(poly)xyloside, et plus particulièrement l'arachidyl(poly)glucoside.

4. Composition selon l'une quelconque des revendications 1 à 3, où le ou les alkylC₈-C₃₀(poly)glycosides sont présents dans des concentrations allant de 0,01 à 10 % en poids, de préférence de 0,1 à 6 % en poids, et plus préférentiellement de 0,5 à 4 % en poids, par rapport au poids total de la composition.

5. Composition selon l'une quelconque des revendications 1 à 4, comprenant en outre au moins un alcool gras en C8-40, de préférence choisi parmi l'alcool cétylique, l'alcool stéarylique, l'alcool arachidylique, l'alcool béhénylique et leurs mélanges, et plus préférentiellement parmi l'alcool arachidylique, l'alcool béhénylique et leurs mélanges.

6. Composition selon la revendication 5, où le ou les alcools gras sont présents dans des concentrations allant de 0,01 % à 15 % en poids, de préférence de 0,1 % à 10 % en poids, et plus préférentiellement de 0,5 % à 5 % en poids, par rapport au poids total de la composition.

7. Composition selon l'une quelconque des revendications précédentes, où le système photoprotecteur est constitué par un ou plusieurs filtres organiques hydrophiles, lipophiles ou insolubles et/ou inorganiques notamment un ou plusieurs pigments minéraux.

8. Composition selon la revendication 7, où les filtres UV organiques sont choisis parmi les dérivés cinnamiques ; les anthranilates ; les dérivés salicyliques ; les dérivés de dibenzoylméthane ; les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'a-alkylstyrène ; les 4,4-diarylbutadiènes ; les dérivés de mérocyanine et leurs mélanges.

9. Composition selon l'une quelconque des revendications 7 ou 8, où les filtres UV organiques sont choisis parmi :
Ethylhexyl Methoxycinnamate,
Ethylhexyl Salicylate,
Homosalate,
Butyl Methoxydibenzoylmethane,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle,
4-Methylbenzylidene camphor,
Terephthalylidene Dicamphor Sulfonic Acid,
Disodium Phenyl Dibenzimidazole Tetra-sulfonate,
Méthylène bis-Benzotriazolyl Tetramethylbutylphénol,
Bis-Ethylhexyloxyphenol Methoxyphenyl Triazine,
Ethylhexyl triazone,
Diethylhexyl Butamido Triazone,
la 2,4,6-tris(4'-amino benzalmalonate de dinéopentyle)-s-triazine,
la 2,4,6-tris-(4'-amino benzalmalonate de diisobutyle)-s- triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine,
la 2,4,6-tris(terphenyl)-1,3,5-triazine,
Drometrizole Trisiloxane,
Polysilicone-15,
1,1-dicarboxy (2,2'-diméthyl-propyl)-4,4-diphénylbutadiène,
2,4-bis-[5-1(diméthylpropyl)benzoxazol-2-yl-(4-phenyl)-imino]-6-(2-ethylhexyl)-imino-1,3,5-triazine,
et leurs mélanges.

10. Composition selon la revendication 7, où les filtres UV inorganiques sont des pigments d'oxyde métallique, enrobés ou non.

11. Utilisation d'un ester d'acide carboxylique tel que défini dans la revendication 1 pour augmenter le facteur de protection solaire (SPF) d'une composition comprenant dans un milieu cosmétiquement acceptable au moins un système photoprotecteur capable de filtrer le rayonnement UV tel que défini dans l'une des revendications précédentes.

12. Procédé cosmétique non thérapeutique de soin et/ou de maquillage d'une matière kératinique comprenant l'application sur la surface de ladite matière kératinique d'une composition telle que définie dans l'une quelconque des revendications 1 à 10.

13. Procédé cosmétique non thérapeutique pour limiter l'assombrissement de la peau et/ou améliorer la couleur et/ou l'homogénéité du teint comprenant l'application sur la surface de la matière kératinique d'une composition telle que définie dans l'une quelconque des revendications 1 à 10.

14. Procédé cosmétique non thérapeutique pour prévenir et/ou traiter les signes du vieillissement d'une matière kératinique comprenant l'application sur la surface de la matière kératinique d'une composition telle que définie dans l'une quelconque des revendications 1 à 10.

## Patentansprüche

1. Zusammensetzung, insbesondere kosmetische Zusammensetzung, **dadurch gekennzeichnet, dass** sie in einem Medium, insbesondere einem kosmetisch unbedenklichen Medium, Folgendes umfasst:
(a) mindestens ein Lichtschutzsystem, das zum Filtern von UV-Strahlung fähig ist;
(b) mindestens einen Ester einer Carbonsäure mit mindestens 12 Kohlenstoffatomen, wobei der Ester mindestens 24 Kohlenstoffatome umfasst;
(c) mindestens ein Alkyl-C₈-C₃₀-(poly)glycosid,
wobei der Carbonsäureester bzw. die Carbonsäureester aus Behenylbehenat, Arachidylarachidat und spezieller Behenylbehenat ausgewählt ist bzw. sind.

2. Zusammensetzung nach Anspruch 1, wobei der Carbonsäureester bzw. die Carbonsäureester in Konzentrationen im Bereich von 0,01 bis 10 Gew.-% und noch weiter bevorzugt von 0,5 bis 5 % und noch spezieller von 1 bis 3 %, bezogen auf das Gesamtgewicht der Zusammensetzung, vorliegt bzw. vorliegen.

3. Zusammensetzung nach einem der Ansprüche 1 bis 2, wobei das Alkyl-C₈-C₃₀-(poly)glycosid bzw. die Alkyl-C₈-C₃₀-(poly)glycoside aus Coco (poly) glycosid, Arachidyl(poly)glycosid, Myristyl(poly)glycosid, Cetylstearyl(poly)glycosid, C₁₂-C₂₀-Alkyl(poly)-glycosiden, Isostearyl(poly)glycosid und Octyldodecyl(poly)xylosid und spezieller Arachidyl(poly)glycosid ausgewählt ist bzw. sind.

4. Zusammensetzung nach einem der Ansprüche 1 bis 3, wobei das Alkyl-C₈-C₃₀-(poly)glycosid bzw. die Alkyl-C₈-C₃₀-(poly)glycoside in Konzentrationen im Bereich von 0,01 bis 10 Gew.-%, vorzugsweise von 0,1 bis 6 Gew.-% und weiter bevorzugt von 0,5 bis 4 Gew.-%, bezogen auf das Gesamtgewicht der Zusammensetzung, vorliegt bzw. vorliegen.

5. Zusammensetzung nach einem der Ansprüche 1 bis 4, die außerdem mindestens einen C8-40-Fettalkohol umfasst, der vorzugsweise aus Cetylalkohol, Stearylalkohol, Arachidylalkohol, Behenylalkohol und Mischungen davon und weiter bevorzugt aus Arachidylalkohol, Behenylalkohol und Mischungen davon ausgewählt ist.

6. Zusammensetzung nach Anspruch 5, wobei der Fettalkohol bzw. die Fettalkohole in Konzentrationen im Bereich von 0,01 bis 15 Gew.-%, vorzugsweise von 0,1 bis 10 Gew.-% und weiter bevorzugt von 0,5 bis 5 Gew.-%, bezogen auf das Gesamtgewicht der Zusammensetzung, vorliegt bzw. vorliegen.

7. Zusammensetzung nach einem der vorhergehenden Ansprüche, wobei das Lichtschutzsystem aus einem oder mehreren hydrophilen, lipophilen oder unlöslichen organischen Filtern und/oder anorganischen Filtern, insbesondere einem oder mehreren mineralischen Pigmenten, besteht.

8. Zusammensetzung nach Anspruch 7, wobei die organischen UV-Filter aus Zimtsäurederivaten; Anthranilaten; Salicylsäurederivaten, Dibenzoylmethanderivaten; Campherderivaten; Benzophenonderivaten; β,β'-Diphenylacrylatderivaten; Triazinderivaten; Benzotriazolderivaten; Benzalmalonatderivaten; Benzimidazolderivaten; Imidazolinen; Bisbenzoazolylderivaten; p-Aminobenzoesäurederivaten (PABA-Derivaten); Methylenbis(hydroxy-phenylbenzotriazol)derivaten; Benzoxazolderivaten; Filterpolymeren und Filtersilikonen; von α-Alkylstyrol abgeleiteten Dimeren; 4,4-Diarylbutadienen; Merocyaninderivaten und Mischungen davon ausgewählt sind.

9. Zusammensetzung nach einem der Ansprüche 7 oder 8, wobei die organischen UV-Filter aus:
Ethylhexyl Methoxycinnamate,
Ethylhexyl Salicylate,
Homosalate,
Butyl Methoxydibenzoylmethane,
Octocrylene,
Phenylbenzimidazole Sulfonic Acid,
Benzophenone-3,
Benzophenone-4,
Benzophenone-5,
2-(4-Diethylamino-2-hydroxybenzoyl)benzoesäure-n-hexylester,
4-Methylbenzylidene Camphor,
Terephthalylidene Dicamphor Sulfonic Acid,
Disodium Phenyl Dibenzimidazole Tetra-sulfonate,
Methylenbis(benzotriazolyl)tetramethylbutylphenol,
Bis-Ethylhexyloxyphenol Methoxyphenyl Triazine,
Ethylhexyl Triazone,
Diethylhexyl Butamido Triazone,
2,4,6-Tris(4-aminobenzalmalonsäuredineopentylester)-s-triazin,
2,4,6-Tris(4-aminobenzalmalonsäurediisobutylester)-s-triazin,
2,4-Bis(4-aminobenzalmalonsäuredineopentylester)-6-(4-aminobenzoesäure-n-butylester)-s-triazin,
2,4,6-Tris(biphenyl-4-yl)-1,3,5-triazin,
2,4,6-Tris(terphenyl)-1,3,5-triazin, Drometrizole Trisiloxane,
Polysilicone-15,
1,1-Dicarboxy(2,2'-dimethylpropyl)-4,4-diphenylbutadien,
2,4-Bis[5-1(dimethylpropyl)benzoxazol-2-yl(4-phenyl)imino]-6-(2-ethylhexyl)imino-1,3,5-triazin und Mischungen davon
ausgewählt sind.

10. Zusammensetzung nach Anspruch 7, wobei es sich bei den anorganischen UV-Filtern um beschichtete oder unbeschichtete Metalloxidpigmente handelt.

11. Verwendung eines Carbonsäureesters gemäß Anspruch 1 zur Erhöhung des Lichtschutzfaktors (LSF) einer Zusammensetzung, die in einem kosmetisch unbedenklichen Medium mindestens ein Lichtschutzsystem, das zum Filtern von UV-Strahlung fähig ist, gemäß einem der vorhergehenden Ansprüche umfasst.

12. Nichttherapeutisches kosmetisches Verfahren zum Pflegen und/oder Schminken eines Keratinmaterials, bei dem man auf die Oberfläche des Keratinmaterials eine Zusammensetzung gemäß einem der Ansprüche 1 bis 10 aufbringt.

13. Nichttherapeutisches kosmetisches Verfahren zum Einschränken des Dunkelwerdens der Haut und/oder Verbessern der Farbe und/oder Homogenität des Teints, bei dem man auf die Oberfläche des Keratinmaterials eine Zusammensetzung gemäß einem der Ansprüche 1 bis 10 aufbringt.

14. Nichttherapeutisches kosmetisches Verfahren zum Verhindern und/oder Behandeln von Anzeichen von Alterung eines Keratinmaterials, bei dem man auf die Oberfläche des Keratinmaterials eine Zusammensetzung gemäß einem der Ansprüche 1 bis 10 aufbringt.

## Claims

1. Composition, notably a cosmetic composition, **characterized in that** it comprises, notably in a cosmetically acceptable medium:
(a) at least one photoprotective system capable of screening out UV radiation;
(b) at least one carboxylic acid ester comprising at least 12 carbon atoms, said ester comprising at least 24 carbon atoms;
(c) at least one C₈-C₃₀alkyl(poly)glycoside,
the carboxylic acid ester(s) being chosen from behenyl behenate and arachidyl arachidate, and more particularly behenyl behenate.

2. Composition according to Claim 1, in which the carboxylic acid ester(s) are present in concentrations ranging from 0.01% to 10% by weight, more preferentially from 0.5% to 5% by weight and even more particularly from 1% to 3% relative to the total weight of the composition.

3. Composition according to either of Claims 1 and 2, in which the C₈-C₃₀alkyl(poly)glycosides are chosen from cocoyl(poly)glucoside, arachidyl(poly)glucoside, myristyl(poly)glucoside, cetylstearyl(poly)glucoside, C₁₂-C₂₀alkyl(poly) glucosides, isostearyl (poly) glucoside and octyldodecyl(poly)xyloside, and more particularly arachidyl(poly)glucoside.

4. Composition according to any one of Claims 1 to 3, in which the C₈-C₃₀alkyl(poly)glycoside (s) are present in concentrations ranging from 0.01% to 10% by weight, preferably from 0.1% to 6% by weight and more preferentially from 0.5% to 4% by weight relative to the total weight of the composition.

5. Composition according to any one of Claims 1 to 4, also comprising at least one C8-40 fatty alcohol, preferably chosen from cetyl alcohol, stearyl alcohol, arachidyl alcohol and behenyl alcohol, and mixtures thereof, and more preferentially from arachidyl alcohol and behenyl alcohol, and mixtures thereof.

6. Composition according to Claim 5, in which the fatty alcohol(s) are present in concentrations ranging from 0.01% to 15% by weight, preferably from 0.1% to 10% and more preferentially from 0.5% to 5% by weight relative to the total weight of the composition.

7. Composition according to any one of the preceding claims, in which the photoprotective system is formed from one or more hydrophilic, lipophilic or insoluble organic screening agents and/or mineral screening agents, notably one or more mineral pigments.

8. Composition according to Claim 7, in which the organic UV-screening agents are chosen from cinnamic derivatives; anthranilates; salicylic derivatives; dibenzoylmethane derivatives; camphor derivatives; benzophenone derivatives; β,β-diphenylacrylate derivatives; triazine derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; α-alkylstyrene-based dimers; 4,4-diarylbutadienes; merocyanin derivatives; and mixtures thereof.

9. Composition according to either of Claims 7 and 8, in which the organic UV-screening agents are chosen from:
Ethylhexyl methoxycinnamate,
Ethylhexyl salicylate,
Homosalate,
Butylmethoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Bis(ethylhexyloxyphenol)methoxyphenyltriazine, Ethylhexyl triazone,
Diethylhexyl butamidotriazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(dineopentyl 4'-aminobenzalmalonate)-6-(n-butyl 4'-aminobenzoate)-s-triazine,
2,4,6-Tris(biphenyl-4-yl)-1,3,5-triazine, 2,4,6-Tris(terphenyl)-1,3,5-triazine,
Drometrizole trisiloxane,
Polysilicone-15,
1,1-Dicarboxy(2,2'-dimethylpropyl)-4,4-diphenylbutadiene;
2,4-Bis[5-(1-dimethylpropyl)benzoxazol-2-yl-(4-phenyl)imino]-6-(2-ethylhexyl)imino-1,3,5-triazine,
and mixtures thereof.

10. Composition according to Claim 7, in which the mineral UV-screening agents are coated or uncoated metal oxide pigments.

11. Use of a carboxylic acid ester as defined in Claim 1 for increasing the sun protection factor (SPF) of a composition comprising, in a cosmetically acceptable medium, at least one photoprotective system capable of screening out UV radiation as defined in any one of the preceding claims.

12. Non-therapeutic cosmetic process for caring for and/or making up a keratin material, comprising the application, to the surface of said keratin material, of a composition as defined in any one of Claims 1 to 10.

13. Non-therapeutic cosmetic process for limiting darkening of the skin and/or improving the colour and/or uniformity of the complexion, comprising the application, to the surface of the keratin material, of a composition as defined in any one of Claims 1 to 10.

14. Non-therapeutic cosmetic process for preventing and/or treating the signs of ageing of a keratin material, comprising the application, to the surface of the keratin material, of a composition as defined in any one of Claims 1 to 10.
